# EUROPEAN PATENT APPLICATION

(11) **EP 1 952 806 A1**
(43) Date of publication of application: **06.08.2008**
(21) Application number: 07002229.8
(22) Date of filing: 01.02.2007
(51) Int. Cl.: A61K 9/14, A61K 9/20, A61K 31/4184, A61K 31/54

(54) **Process for the preparation of adsorbates of candesartan**

(71) Applicant: Helm AG, 20097 Hamburg (DE)
(72) Inventor: Glänzer, Klaus, Dr., 22337 Hamburg (DE); Löffler, Uwe, Dr., 25436 Tornesch (DE); Stritzke, Katja, Dr., 20259 Hamburg (DE)
(74) Representative: Becker Kurig Straus

(57) **Abstract**

The present invention relates to a process for the preparation of adsorbates of candesartan cilexetil and/or its solvates or hydrates. In a particularly preferred embodiment, the adsorbates according to the present invention contain the active ingredient or one of its pharmaceutically acceptable salts and derivatives and/or their solvates or hydrates in an amorphous form. The invention further relates to candesartan cilexetil adsorbates that are obtainable by said process, as well as pharmaceutical formulations prepared employing said candesartan cilexetil adsorbates. Preferred drug formulations according to the invention are tablets, capsules, pellets, and granules prepared with the usual, pharmaceutically acceptable adjuvants in ways known per se. Particularly preferred according to the invention are tablets rapidly releasing the active ingredient that are obtained by direct compressing of the candesartan cilexetil adsorbates according to the present invention with or without excipients.

## Description

### BACKGROUND OF THE INVENTION

### 1. Field of the Invention

The present invention relates to a novel process for the preparation of adsorbates of candesartan cilexetil and/or its solvates or hydrates. In a particularly preferred embodiment, the adsorbates according to the present invention contain the active ingredient or one of its pharmaceutically acceptable salts and derivatives and/or their solvates or hydrates in an amorphous form. The invention further relates to candesartan cilexetil adsorbates that are obtainable by said process, as well as pharmaceutical formulations prepared employing said candesartan cilexetil adsorbates. Preferred drug formulations according to the invention are tablets, capsules, pellets, and granules prepared with the usual, pharmaceutically acceptable adjuvants in ways known per se. Particularly preferred according to the invention are tablets rapidly releasing the active ingredient that are obtained by direct compressing of the candesartan cilexetil adsorbates according to the present invention with or without excipients.

### 2. Description of Related Art

The active ingredient having the chemical name of (±)-1-(Cyclohexyloxycarbonyloxy)ethyl 2-ethoxy-1-[[2'-(1H-tetrazol-5-yl)biphenyl-4-yl]methyl]-benzimidazole-7-carboxylate, also known by the INN as candesartan cilexetil, can be represented by the following structural formula:

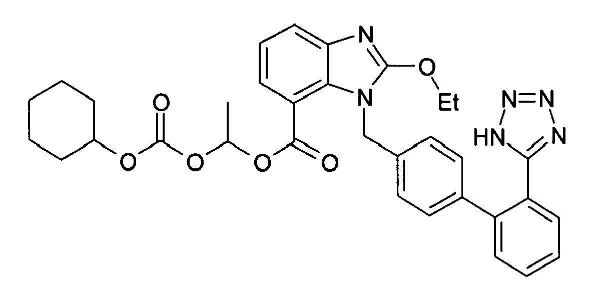

Candesartan cilexetil belongs to the class of non-peptide angiotensin-II receptor antagonists having a very high selectivity for the AT1 receptors. Candesartan cilexetil is a pro-drug and is hydrolyzed to candesartan during absorption from the gastrointestinal tract. It thus falls in the class of benzimidazole-7-carboxylic acid derivatives. These agents exhibit a strong and more effective hypotensive action and are less likely to cause coughing as a side effect as compared to other classes of ACE inhibitors. With a common daily dose of 2 to 32 mg, candesartan cilexetil is used as a single-substance preparation (mono-preparation), or in combination with the diuretic hydrochlorothiazide, for the treatment of cardiovascular diseases. As an AT1 receptor antagonist, candesartan cilexetil more particularly inhibits the rise of blood pressure caused by angiotensin II, suppresses angiotensin-II-induced aldosterone secretion, and lowers angiotensin-II-induced liquid uptake *(see, for instance,* Arzneimittelwirkung: Lehrbuch der Pharmakologie und Toxikologie, E. Mutschler, G. Geisslinger, H.K. Kroemer, M Schäfer-Korting, editors, 8<th > edition, Wissenschaftliche Verlagsgesellschaft mbH Stuttgart, 2001*).*

Candesartan cilexetil and its pharmaceutically acceptable salts and processes for their preparation are described in EP 0 459136, reference example 2-8. The preparation of candesartan cilexetil in its C-type crystalline form and in its amorphous form is also described in EP 0 459136. Candesartan cilexetil of the C-type crystalline form (Form C) is obtained by crystallization from lower alcohols (methanol, ethanol), mixtures of a lower alcohol and water, and mixtures of a lower alkyl ketone (like acetone) and water. The amorphous candesartan cilexetil can be purified by column chromatography of crude candesartan cilexetil and evaporation of the solvent. However, the amorphous powder is said to be unstable to heat and impractical in production (experimantal example 1).

Two crystalline forms of candesartan cilexetil, referred to as form I and form II, are described in Chem. Pharm. Bull. 47 (2), 182-186 (1999).

In WO 2004/085426 candesartan cilexetil 1,4-dioxane solvate, prepared by dissolving candesartan cilexetil in dioxane and crystallization of the 1,4-dioxane solvate at a temperature of 5-15°C, is disclosed. This application further comprises candesartan cilexetil form III crystallized from toluene, and candesartan cilexetil form IV which is crystallized from a mixture of methyl-t-butyl-ether (MTBE) and methanol. The disclosed polymorphic forms of this application have been characterized by their x-ray diffraction patterns.

WO 2005/077941 discloses a large variety of polymorphic forms of candesartan cilexetil and its solvates and their preparation processes. Form II, III, IV, V, VI, VII, VIII, IX, X, XI, XIII, XIV, XIV-1, XV, XVI, XVII, XVIII, XIX, XX, XXI, XXII, XXIII, and amorphous candesartan cilexetil are part of the application.

WO 2005/123721 describes an amorphous form of candesartan cilexetil, which is prepared by spray drying, having less than about 600 ppm residual dichloromethane solvent.

In the solid state, pure candesartan cilexetil is stable against moderate heat, moisture and light. Candesartan cilexetil has low solubility in water because it is hydrophobic in nature. The low bioavailability of hydrophobic drugs with extremely low water solubility can be a serious problem. When candesartan is formulated into tablets with other ingredients in order to improve the solubility and dissolution characteristics, it has been observed that the content of the active ingredient decreases over time. Different approaches have been taken to achieve a desired level of drug solubility and dissolution rate.

EP 0 546358 discloses that the reduction in the content of active candesartan cilexetil with the lapse of time in pharmaceutical compositions can be reduced by incorporating oily substances having a low melting point in these compositions. The oily substances are incorporated into the active component to form a stable composition in which decomposition with time caused by compression is suppressed.

In EP 1 441705 a process is disclosed for the preparation of stable solid or semisolid formulations starting from liquid active principles which undergo prompt or modified release from the system. The active principles are stabilized by the use of amphiphilic and/or lipophilic matrices characterized by melting temperatures ranging from 30°C to 90°C and by being solids at room temperatures to at least 25°C.

WO 2004/071494 deals with pharmaceutical compositions containing biologically active compounds with low water solubility complexed with water insoluble polymers to enhance solubility in aqueous media. The therapeutic agent and lipophilic polymer are homogeneously dispersed in a water-miscible organic solvent and precipitated by the addition of water or removal of the solvent.

In WO 2005/070398 stable pharmaceutical compositions for oral administration including candesartan cilexetil and one or more co-solvents are provided. The co-solvent is selected from propylene glycol, polyethylene alcohol, ethanol, glycerin, propylene glycol esters, or polyethylene glycol esters. Optionally the composition may comprise a fatty acid ester like glycerol stearate, glycerol palmitate, glyceryl caprate, glyceryl caprylate, glycerol oleate, glycerol linoleate, or glyceryllauropalmitoleate.

WO 2005/079751 discloses a pharmaceutical composition that includes candesartan cilexetil and one or more fatty substances which are present at concentrations of about 0.5% up to about 10% w/w. The fatty substances may be lipids and phospholipids.

In WO 2005/084648 pharmaceutical compositions including candesartan cilexetil and one or more water-soluble polymers are described. The pharmaceutical composition contains a water-soluble polymer selected from polyvinyl alcohol, maltodextrin, xanthan gum, polyvinylpyrrolidone, hydroxypropyl cellulose, hydroxypropyl methyl cellulose, hydroxyethyl cellulose, sodium carboxymethylcellulose, calcium carboxymethylcellulose, methyl cellulose, tragacanth gum, agar, gellan gum, karaya gum, alginic acids, pectins, or pregelatinized starch.

Finally WO 2005/123720 provides candesartan cilexetil having a particle size D_{0.9} of about 25 microns or less. According to the application the pharmaceutical solid composition comprising candesartan cilexetil of reduced particle size exhibits a significantly improved pharmacokinetic profile and good bioavailability. The small particle sized candesartan cilexetil is prepared by specific crystallization, grinding, milling, jet milling, media milling or pulverization. The inventors state that a pharmaceutical solid formulation containing candesartan cilexetil with D_{0.9} values of about 70 microns down to about 40 microns exhibits a pharmacokinetic profile which is not predictable.

None of the above mentioned formulations of candesartan cilexetil is completely satisfactory because they all require additional stabilizing excipients like oily substances, co-solvents, polymers, amphiphilic or lipophilic matrices, or/and fatty acid substances. Another formulation procedure demands time and energy consuming processes to reduce the particle size of candesartan cilexetil. Known processes for the preparation of pharmaceutical formulations containing amorphous candesartan cilexetil, which is said to be unstable, require at least a comparable technical effort and are time and cost intensive, if they can at all be realized. Thus, it is an object of the present invention to provide candesartan cilexetil formulations with an optimized drug performance in a short, efficient and simple process. The solid pharmaceutical formulations obtained according to the present invention as adsorbates do not need any additional stabilizing agents and may be prepared starting from the amorphous or any polymorphic form of candesartan cilexetil and its hydrates or solvates in any particle size. The solid pharmaceutical formulations of these candesartan cilexetil adsorbates exhibit improved dissolution characteristics.

### BRIEF SUMMARY OF THE INVENTION

The object of the present invention is to develop a simple and economical process for the preparation of candesartan cilexetil powder systems that can be used immediately for manufacturing pharmaceutical formulations, this process not being restricted, however, to a particularly preferred morphology (amorphous, crystalline polymorphs) of the active ingredient or its hydrates or solvates, and avoiding the disadvantages exhibited by previous products discussed above.

### BRIEF DESCRIPTION OF THE DRAWINGS

FIG. 1 shows the powder X-ray diffraction pattern of candesartan cilexetil in its crystalline form C.
FIG. 2 shows the powder X-ray diffraction pattern of a candesartan cilexetil-mannitol adsorbate according to example 2 of the present invention.
FIG. 3 shows the powder X-ray diffraction pattern of a candesartan cilexetil-lactose adsorbate according to example 1 a of the present invention.
FIG. 4 shows the powder X-ray diffraction pattern of α-lactose
FIG. 5 shows the powder X-ray diffraction pattern of a candesartan cilexetil-lactose adsorbate (example 1a) after subtraction of the powder X-ray diffraction pattern of the α-lactose employed in the preparation.
FIG. 6 shows the powder X-ray diffraction pattern of a candesartan cilexetil-lactose adsorbate according to example 1b of the present invention
FIG. 7 shows the powder X-ray diffraction pattern of a candesartan cilexetil-lactose adsorbate according to example 1c of the present invention.
FIG. 8 shows the dissolution profiles of Atacand®, of the formulation 2a prepared according to example 1a containing candesartan cilexetil lactose adsorbate, and of the reference formulation 2b, containing crystalline candesartan cilexetil in its crystalline Form C.

### DETAILED DESCRIPTION OF THE INVENTION

The present invention relates to a process for the preparation of adsorbates of candesartan cilexetil and/or of its solvates or hydrates according to which one starts from a solution of candesartan cilexetil and/or its solvates or hydrates in at least one organic solvent., disperses the adsorbing material in it, and removes the solvent, which can more particularly be achieved by drying. In a preferred embodiment of the invention, said candesartan cilexetil adsorbates contain the active ingredient and/or their solvates or hydrates in an amorphous form.

The invention further relates to adsorbates of candesartan cilexetil and/or its solvates or hydrates that are obtainable by said process.

It also relates to pharmaceutical formulations containing these novel candesartan cilexetil adsorbates. Where applicable, the pharmaceutical formulations may contain further adjuvants and may be converted to the desired drug delivery formulation. Tablets produced by direct compressing which rapidly release the active ingredient are particularly preferred.

The pharmaceutical formulations according to the invention which contain candesartan cilexetil adsorbates as the AT1 receptor antagonist can be employed to treat diseases that can for instance be described as follows:
(a) Hypertension, cardiac insufficiency, renal failure, particularly chronic renal failure, restenosis after percutaneous transluminal angioplasty, and restenosis after coronary arterial bypass surgery,
(b) arteriosclerosis, insulin resistance and syndrome X, diabetes mellitus type 2, obesity, nephropathy, renal failure, for instance chronic renal failure, hypothyreosis, the condition after cardiac infarction, coronary cardiopathy, age-related hypertension, familial dyslipidemic hypertension, all the precited diseases in connection with or without hypertension,
(c) endothelial dysfunction in connection with or without hypertension,
(d) hyperlipidemia, hyperlipoproteinemia, arteriosclerosis and hypercholesterinemia,
(e) glaucoma.

Organic solvents in which the active pharmaceutical ingredient can be dissolved are suitable for the process according to the invention for the preparation of candesartan cilexetil adsorbates.

The organic solvents are selected from the group of lower alkanones, alkanols, ethers, esters, aromatic and aliphatic hydrocarbons, aliphatic amides, halogenated hydrocarbons, nitriles, dialkyl sulfoxides as well as mixtures of said solvents. The alkyl moieties of the solvents may contain one to four carbon atoms. The solvents may be used in their anhydrous form or may contain water.

Particularly the solvents are selected from acetone, methyl ethyl ketone, methanol, ethanol, isopropanol, n-propanol, 1-butanol, 2-butanol, t-butanol, diethyl ether, dimethyl ether, tetrahydrofuran, dioxane, diisopropyl ether, methyl acetate, ethyl acetate, propyl acetate, isopropyl acetate, toluene, xylene, mesitylene, petroleum ether, hexane, cyclohexane, heptane, dichloromethane, chloroform, ethylene dichloride, dimethylformamide, dimethyl acetamide, or acetonitrile. More preferable are the solvents acetone, toluene and dichloromethane. The most preferred solvent is acetone.

According to the present invention, those pharmaceutically acceptable adjuvants are used as adsorbing materials which are appropriate for a rapid release of the active ingredient. They generally belong to the class of carbohydrates, such as celluloses and cellulose derivatives, more particularly lactose, starches and starch derivatives, cyclodextrins, polydextroses or mixtures of said substances. Microcrystalline cellulose, lactose, and mannitol are preferred according to the invention. For an improvement of the flow properties, additives containing silica or titania can be used.

The ratio of pharmaceutically active ingredient to adsorbing material according to the invention is in the range from 5:1 to 1:10, a range from 2:1 to 1:2 being particularly preferred.

All common pharmaceutical adjuvants can be used to prepare the pharmaceutical formulations, where tablets are more particularly preferred. As fillers, for example celluloses and cellulose derivatives (for instance microcrystalline cellulose, powdered cellulose, hydroxypropyl cellulose, hydroxypropyl methyl cellulose, methyl cellulose), sugars (for instance lactose, fructose, saccharose, glucose, maltose), sugar alcohols (for instance lactitol, mannitol, sorbitol, xylitol), inorganic fillers (for instance calcium phosphates and calcium sulfates), and starches and starch derivatives (for instance corn starch, potato starch, wheat starch, dextrins, pregelatinized starches) can be used. Beyond that, all other adjuvants known to those skilled in the art from their basic galenic knowledge, such as lubricants, disintegration aids, wetting agents, agents to improve the flow behaviour, other additives, stabilizers, as well as flavours, pigments, and dyes, can be used to prepare the drug formulations according to the invention (those skilled in the art can take the corresponding information, for instance, from: Die Tablette, W. A. Ritschel and A. Bauer-Brandl, 2^{nd} ed., ECV-Editio Cantor publishers, 2002).

Immediate release tablets preferably comprise:
1 to 99 wt.-%, preferably 1 to 75 wt.-% and more preferably 1 to 40 wt.-% of candesartan cilexetil adsorbate,
0 to 50 wt.-%, preferably 5 to 25 wt.-% and more preferably 8 to 20 wt.-% disintegrantion aids,
0 to 50 wt.-%, preferably 1 to 25 wt.-% and more preferably 5 to 20 wt.-% binder,
0 to 95 % wt.-%, preferably 1 to 85 wt.-% and more preferably 20 to 80 wt.-% filler, and
0.1 to 10 wt.-%, preferably 0.1 to 7,5 wt.-% and more preferably 0.1 to 3 wt.-% lubricant.

Additionally, the pharmaceutical formulation may comprise 0.5 to 25 wt.-%, preferably 2 to 20 wt.-%, and more preferably 5-10 wt.-% of hydrochlorothiazide (HCT).

The process according to the invention yields surprisingly stable, homogeneous adsorbates of candesartan cilexetil preferably free of any crystalline form of the active agent. These candesartan cilexetil adsorbates are used as active pharmaceutical ingredients in the preparations according to the invention.

Within this invention, the respective hydrates, or solvates, of candesartan cilexetil which may originate more particularly in the solution obtained at the end of the synthetic route to the active ingredient, can also be employed, such that isolation of the active ingredient is not necessary and can be left out.

According to the invention, a process has now been found which, starting from a solution of candesartan cilexetil in an organic solvent, leads to adsorbates of the active ingredient that can be processed directly in the drug formulation.

In an embodiment of the invention, the solution of the candesartan cilexetil active ingredient can in principle be prepared by dissolving the active ingredient in a suitable organic solvent; however, it is more advantageous to directly use a solution of the active ingredient resulting from the synthesis, without isolation of the candesartan cilexetil.

Candesartan cilexetil can for instance be prepared according to EP 0 459136 B1, reference example 2-8, but omitting the recrystallization steps involving, e.g., a dissolution in ethanol and cooling the solution in ice. Instead, the adsorbing material is dispersed in the solution of candesartan cilexetil - before or after the chromatographic purification -, and subsequently the solvent is removed in a drying process. The kind of organic solvent used then is determined, in any given case, by the choice of solvent for the final step of the synthesis of the active ingredient.

To this solution of candesartan cilexetil in an organic solvent, a pharmaceutically acceptable adjuvant that is insoluble or poorly soluble in this solvent is added as the adsorbing material, well wetted, and immediately thereafter the solvent is removed by drying. The drying process can be promoted by heating, applying a vacuum, sublimation drying, or also by spray drying. Advantageously, it is conducted in such a way that appropriate mechanical action (e.g., rotating, tumbling, or stirring motion) yields a uniform particle distribution of the product.

The solvent can be recovered by working in a closed system, and reused for a subsequent process. Adsorbates prepared by the process described can be employed directly in further drug formulations to produce tablets, capsules, pellets, or granules, preferably in further processing by a direct compressing process.

Optionally, the adsorbates or drug formulations thus obtained can be further provided with coatings of pharmaceutical polymethacrylates such as Eudragit(R) films, or of methyl celluloses, ethyl celluloses, hydroxypropyl methyl celluloses, cellulose acetate phthalates and/or shellac in order to meet requirements for specific applications, e.g., controlled release of the active ingredient and/or taste masking. Those skilled in the art of pharmaceutics have a range of technical possibilities to do this.

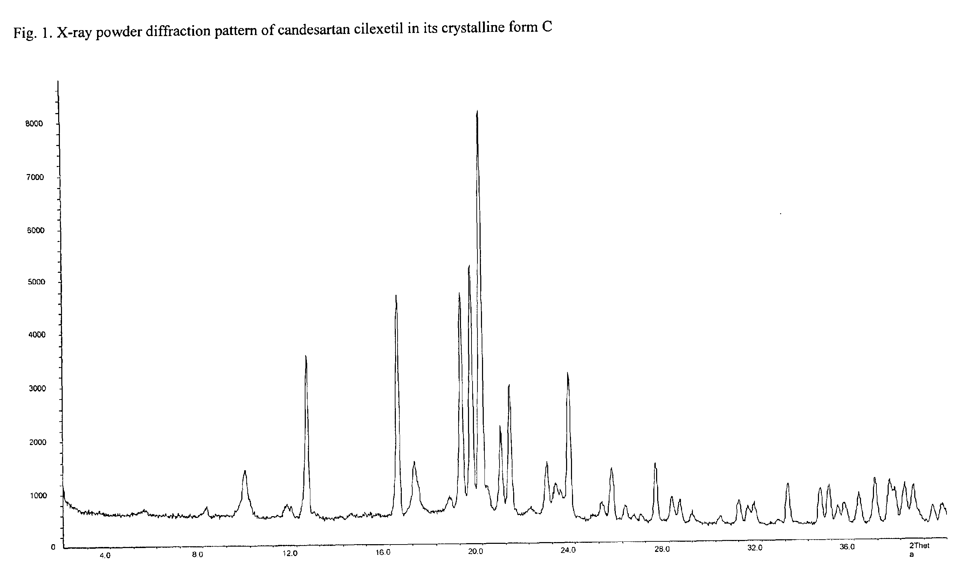

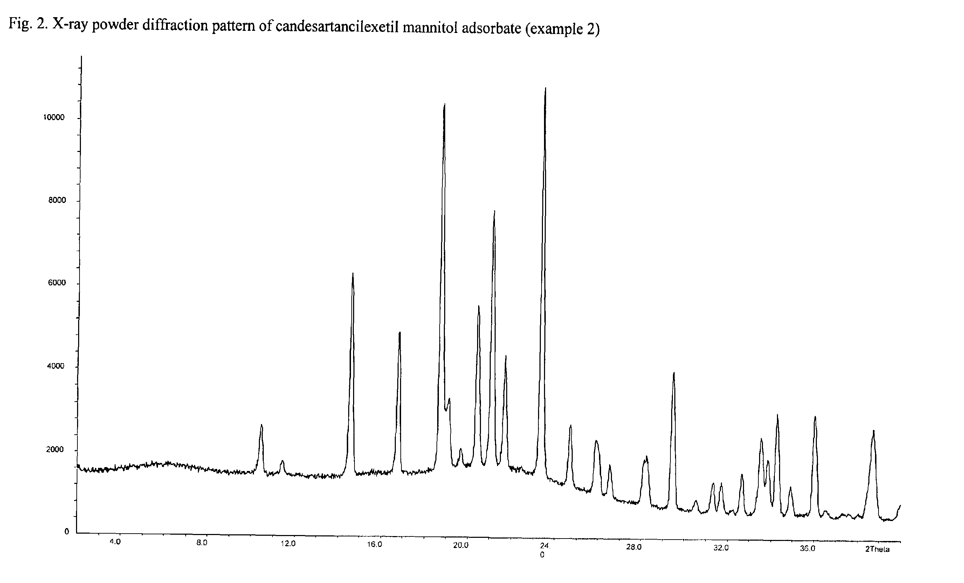

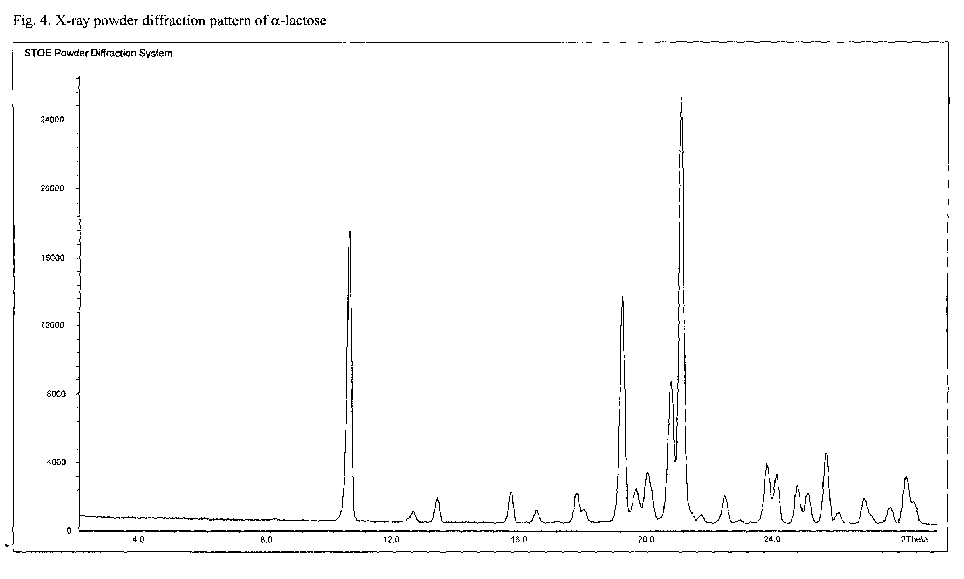

Surprisingly it has been found that adsorbates prepared by the process according to the present invention contain the active ingredient in the homogeneous distribution required for drugs, and that they release it without limitations. The adsorbates prepared by this process have the active ingredient bound in such a way that crystal structures typical of the active ingredient are only poorly developed or preferably not at all. This could be demonstrated by X-ray diffraction (see Fig. 2 to 5). For comparison, Fig. 1 shows a powder X-ray diffraction pattern of the crystalline form C of candesartan cilexetil.

The candesartan cilexetil adsorbates of the present invention may comprise minor amounts of crystalline candesartan cilexetil. Preferably the candesartan cilexetil adsorbates of the invention comprises less than 5 % by weight of crystalline candesartan cilexetil parts, preferably e.g. less than 2 % by weight, based on the total amount of candesartan cilexetil. Most preferably the candesartan cilexetil adsorbates of the present invention comprise substantially no crystalline candesartan cilexetil.

From the observations it appears that part of the active ingredient is first absorbed from solution on the surface of the adsorbent, followed by a growth of the initially adsorbed deposits as the volume of the solvent is subsequently reduced. In both steps of this proposed mechanism, the amorphous state is formed almost exclusively. Upon dissolution of the adsorbate thus formed in water, the adsorbent support is either also dissolved (e. g. lactose, mannitol) or hydrated (e. g. cellulose), such that the active ingredient is readily released.

The characteristic advantageous properties of the product just mentioned are retained when the candesartan cilexetil adsorbates are processed for drug formulations to obtain, e.g., tablets. In particular, direct compressing of the adsorbates does not entail any change in their morphology.

The invention is explained more closely by the following examples and figures, without however limiting thereby the invention.

### EXAMPLES 1 TO 5

Methods of Analysis Used
1. HPLC method for determining the content of active ingredient or sum of all contaminants:
   - Instrument: Aigilent 1100
   - Mobile phase:: Buffer, acetonitrile
   - Column :: Kromasil CN (250mm*4mm, 5µm)
2. Release of active ingredient (dissolution test) according to FDA: USP method II, 50 rpm, medium 0.35 % polysorbate 20 in 0.05 M phosphate buffer of pH 6.5, 900 ml, 37°C
3. The powder X-ray diffraction patterns were recorded as follows:
   - Instrument:: STADI P transmission diffractometer
   Cu K_{α1} radiation (λ, = 1.54056 A), U = 40 kV, I = 30 mA
   Secondary beam monochromator (flat, graphite)
   - Detector:: Scintillation counter
   - Aperture:: 2*8 mm; 0.7 mm; 0.35 mm

   - Linear PSD:: 2 θ = 2° to 35°, 5 s/0.04° in steps
   - Sample:: Powder, reflection mode

### Adsorbate Example 1

### Candesartan cilexetil-Lactose adsorbate (1:1)

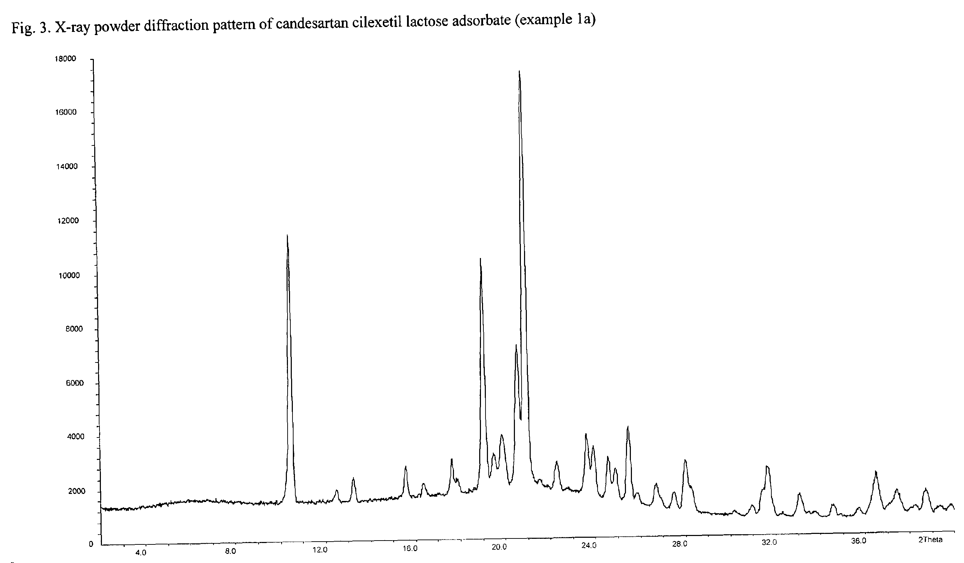

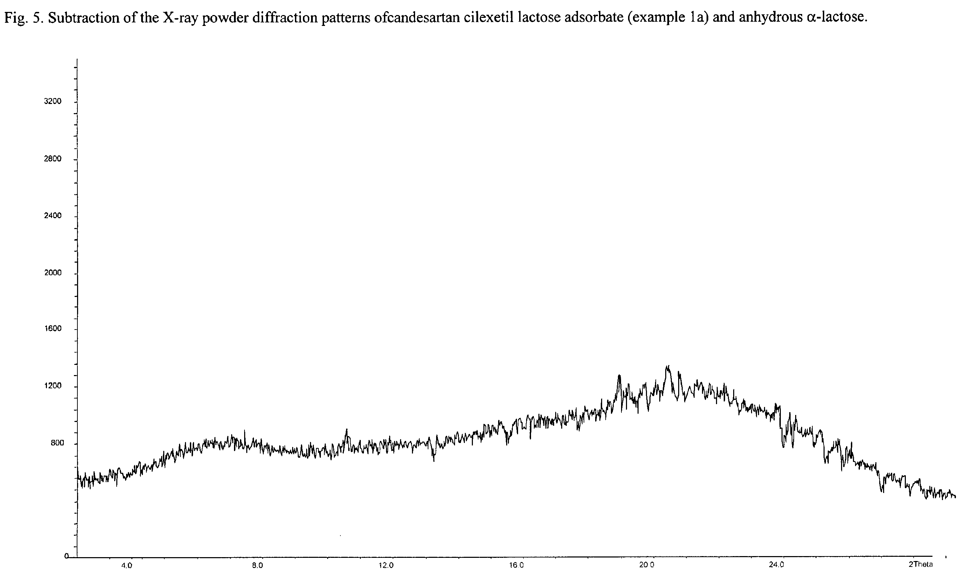

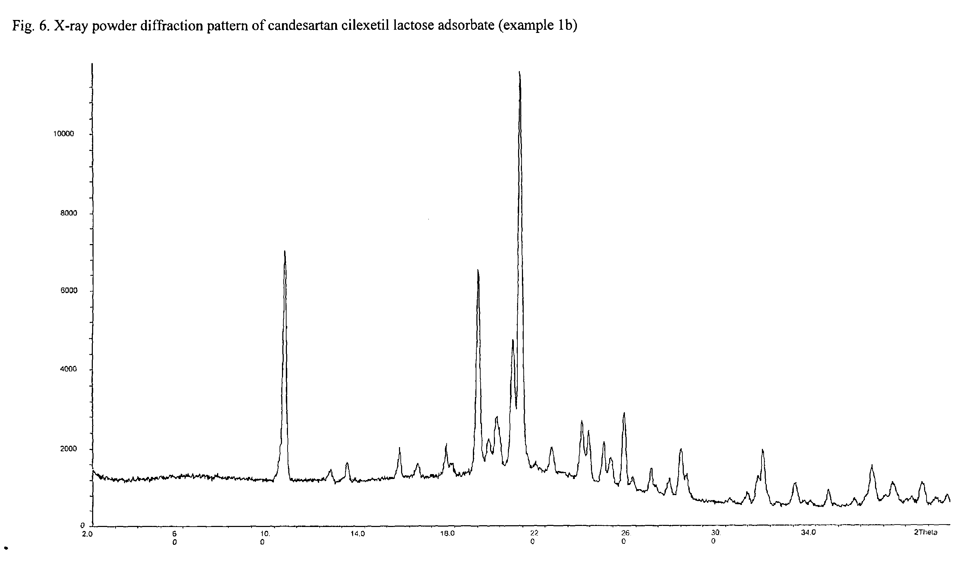

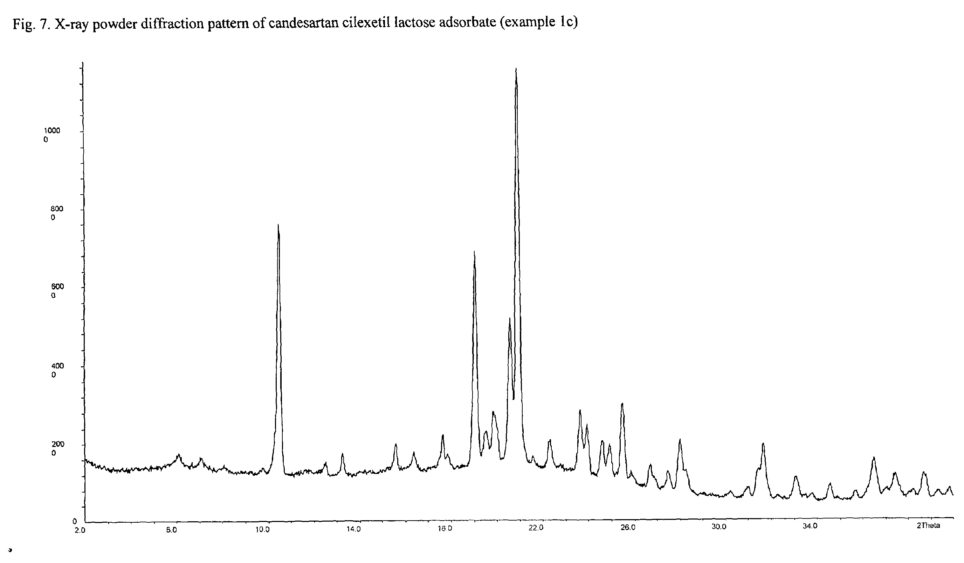

To a solution of candesartan cilexetil (100 mg/ml) in the organic solvent, 100 mg/ml lactose (Lactopress(R), anhydrous) is added and uniformly dispersed. The solvent is removed with continuous agitation in a vacuum (rotatory vaporization) at 40° C. The mixture is finally thoroughly dried to remove any residual solvent.
Theoretical content of active ingredient in the adsorbate: 50%
Used solvents:
a) Acetone: X-ray diffraction pattern: see FIG. 3 and FIG. 5 Dissolution: 73.5 % after 60 min
b) Dichloromethane: X-ray diffraction pattern: see FIG. 6
c) Toluene: X-ray diffraction pattern: see FIG. 7

### Adsorbate Example 2

### Candesartan cilexetil-Mannitol adsorbate (1:1)

To a solution of candesartan cilexetil (100 mg/ml) in acetone, 100 mg/ml mannitol (Mannogem®) is added and uniformly dispersed. The solvent is removed with continuous agitation in a vacuum (rotatory vaporization) at 40° C. The mixture is finally thoroughly dried to remove any residual solvent.
Theoretical content of active ingredient in the adsorbate: 50%
X-ray diffraction pattern: see FIG. 2

### Adsorbate Example 3

### Candesartan cilexetil-microcrystalline cellulose adsorbate (1:1)

To a solution of candesartan cilexetil (100 mg/ml) in acetone, 100 mg/ml mannitol (Mannogem®) is added and uniformly dispersed. The solvent is removed with continuous agitation in a vacuum (rotatory vaporization) at 40° C. The mixture is finally dried thoroughly to remove any residual solvent.

Theoretical content of active ingredient in the adsorbate: 50%

### Adsorbate Example 4

### Candesartan cilexetil-lactose adsorbate (2:1)

To a solution of candesartan cilexetil (100 mg/ml) in acetone, 50 mg/ml lactose (Lactopress(R), anhydrous) is added and uniformly dispersed. The solvent is removed with continuous agitation in a vacuum (rotatory vaporization) at 40° C. The mixture is finally dried thoroughly to remove any residual solvent.
Theoretical content of active ingredient in the adsorbate: 66,7%

### Adsorbate Example 5

### Candesartan cilexetil-lactose adsorbate (1:2)

To a solution of candesartan cilexetil (50 mg/ml) in acetone, 100 mg/ml mannitol (Mannogem®) is added and uniformly dispersed. The solvent is removed with continuous agitation in a vacuum (rotatory vaporization) at 40° C. The mixture is finally thoroughly dried to remove any residual solvent.
Theoretical content of active ingredient in the adsorbate: 33,3 %

### FORMULATIONS 1 TO 4

From the adsorbate, tablets are made by direct compressing in a Korsh EK1 apparatus in the following composition:

### Formulation 1

| *2 mg Candesartan cilexetil immediate release tablet* | |
|---|---|
| Candesartan Cilexetil adsorbate (1:1) | 4 mg |
| Microcrystalline cellulose | 4.8 mg |
| Lactose monohydrate | 46 mg |
| Corn starch | 10 mg |
| Mg stearate | 0.2 mg |
| | Σ 65 mg |

### Formulation 2

| *4 mg Candesartan cilexetil immediate release tablet* | |
|---|---|
| a) Candesartan cilexetil adsorbate (1:1) | 8 mg |
| Microcrystalline cellulose | 9.6 mg |
| Lactose monohydrate | 92 mg |
| Corn starch | 15 mg |
| Kollidon CL | 4.7 mg |
| Mg stearate | 0.4 mg |
| | ∑ 129.7 mg |

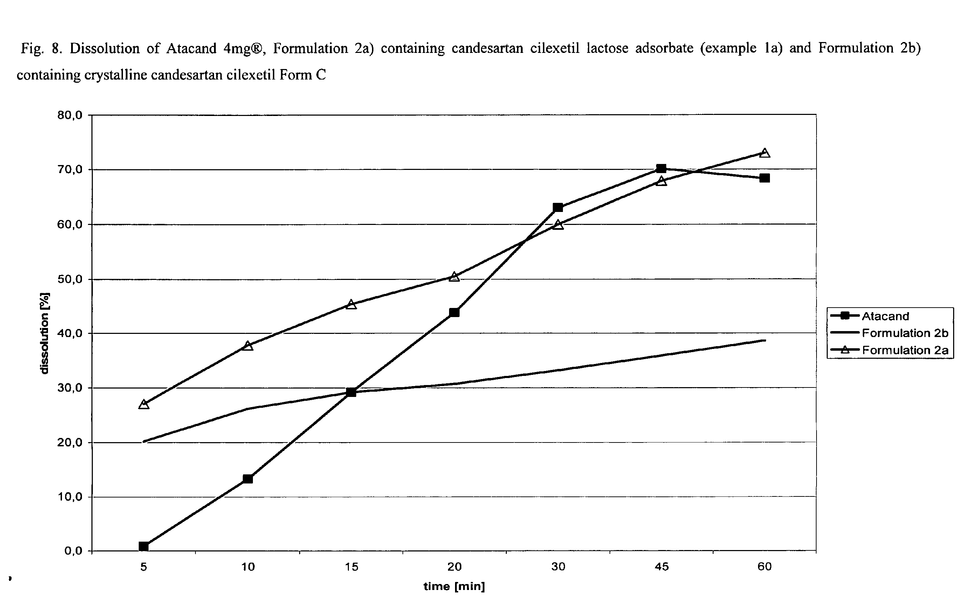

Dissolution of tablets (diameter = 6mm, thickness = 3.3 mm) of formulation 2a) containing candesartan cilexetil lactose adsorbates of example 1a: 73% after 60 min, compare FIG. 8.

### b) Reference formulation containing crystalline candesartan cilexetil of Form C (particle size: 10%< 0.9 µm, 50% < 3.2 µm, 90%< 8.2 µm)

| | |
|---|---|
| Candesartan cilexetil | 4 mg |
| Lactose | 4 mg |
| Microcrystalline cellulose | 9.6 mg |
| Lactose monohydrate | 92 mg |
| Corn starch | 15 mg |
| Kollidon CL | 4.7 mg |
| Mg stearate | 0.4 mg |
| | Σ 129.7 mg |

Dissolution of tablets (diameter= 6mm, thickness= 3.2 mm) of formulation 2b) containing crystalline candesartan cilexetil: 38.6 % after 60 min, compare FIG 7

### Formulation 3

| *4 mg Candesartan cilexetil immediate release tablet* | |
|---|---|
| Candesartan cilexetil adsorbate (1:1) | 8 mg |
| Microcrystalline cellulose | 9.6 mg |
| Lactose monohydrate | 92 mg |
| Corn starch | 15 mg |
| Pregelatinized starch | 4.7 mg |
| Mg stearate | 0.4 mg |
| | Σ 129.7 mg |

Dissolution of tablets of formulation 3 containing candesartan cilexetil lactose adsorbates of example 1: 76.8% after 60 min

### Formulation 4

| *16 mg Candesartan cilexetil immediate release tablet* | |
|---|---|
| Candesartan cilexetil adsorbate (1:1) | 32 mg |
| Microcrystalline cellulose | 5.6 mg |
| Lactose monohydrate | 68 mg |
| Corn starch | 20 mg |
| Mg stearate | 0.4 mg |
| Fe(III) oxide | q.s. |
| | Σ 126 mg |

### Formulation 5

| | |
|---|---|
| Candesartan cilexetil adsorbate (1:1) | 16 mg |
| HCT | 12.5 mg |
| Microcrystalline cellulose | 9.6 mg |
| Lactose monohydrate | 71.5 mg |
| Corn starch | 15 mg |
| Mg stearate | 0.4 mg |
| Starch 1500 | 5 |
| Fe(III) oxide | q.s. |
| | Σ 130 mg |

The tablets thus obtained can be provided with a coating.

## Claims

1. A process for the preparation of adsorbates of candesartan cilexetil and/or of its solvates or hydrates, wherein one starts from a solution of candesartan cilexetil and/or its solvates or hydrates in at least one organic solvent, disperses in it an adsorbing material and removes the solvent.

2. The process according to claim 1, wherein the adsorbates contain the active ingredient, or its solvates or hydrates, in an amorphous form.

3. The process according to claim 2, wherein the weight ratio of active ingredient to adsorbing material is set in the range from 5:1 1 to 1:10, more particularly in the range from 2:1 to 1:2.

4. The process according to claim 1, wherein the weight ratio of active ingredient to adsorbing material is set in the range from 5:1 1 to 1:10, more particularly in the range from 2:1 to 1:2.

5. The process according to claim 1, wherein the absorbing materials are selected in particular from the group of carbohydrates and related polyols, including celluloses, cellulose derivatives, sugars, sugar derivatives, cyclodextrins, starches, starch derivatives, polydextroses, or mixtures thereof.

6. The process according to claim 1, wherein a single organic solvent is used or mixtures of solvents, these organic solvents being selected from the group of lower aliphatic ketones, alkanols, ethers, esters, aromatic and aliphatic hydrocarbons, amides, halogenated hydrocarbons, nitriles, and dialkyl sulfoxides.

7. The process according to claim 6, wherein the solvent is selected from acetone, methyl ethyl ketone, methanol, ethanol, isopropanol, n-propanol, 1-butanol, 2-butanol, t-butanol, diethyl ether, dimethyl ether, tetrahydrofuran, dioxane, diisopropyl ether, methyl acetate, ethyl acetate, n-propyl acetate, isopropyl acetate, toluene, xylene, mesitylene, petroleum ether, hexane, cyclohexane, heptane, dichloromethane, chloroform, ethylene dichloride, dimethyl formamide, dimethyl acetamide, or acetonitrile as well as mixtures of the aforementioned solvents.

8. The process according to claim 6, wherein acetone, toluene or dichloromethane are selected as solvents.

9. The process according to claim 1, wherein a solution of the active ingredient is used which is obtained in the final step of the candesartan cilexetil synthesis.

10. Adsorbates of candesartan cilexetil and its solvates or hydrates obtainable through the process according to claims 1 to 9.

11. Adsorbates of candesartan cilexetil and its solvates or hydrates according to claim 10, wherein the active ingredient or its pharmaceutically acceptable salts or derivatives and their solvates or hydrates are adsorbed in an amorphous form.

12. Adsorbates of candesartan cilexetil and its solvates and hydrates according to claim 10, wherein the adsorbates contain the active ingredient, or a pharmaceutically acceptable salt or other derivative of it, or their solvates or hydrates, in a substantially amorphous or semi-crystalline form.

13. Pharmaceutical formulations with at least one active ingredient and optionally further, pharmaceutically acceptable adjuvants, wherein the formulation contains a candesartan cilexetil adsorbate according to claims 1 to 12 as active ingredient.

14. Pharmaceutical formulations according to claim 13, wherein the formulation is in the formulation of tablets, capsules, pellets, and granules that are obtainable in a known way with common, pharmaceutically acceptable adjuvants.

15. Pharmaceutical formulations according to claims 13 and 14 in the form of tablets made by direct compressing of the components and **characterized by** a rapid release of the active ingredient.

16. Pharmaceutical formulations according to claims 13 to 15, wherein the portion of binder in the total mixture used to prepare the drug is between 0 and 50 wt.%.

17. Pharmaceutical preparations according to claims 13 to 16, wherein the portion of fillers and adjuvants in the total mixture of the drug preparation is from 1 to 99% by weight, preferably from 60 to 99% by weight.

18. Pharmaceutical preparations according to claims 13 to 17, wherein the adsorbates contain the active ingredient or a pharmaceutically acceptable salt or derivative of it, or their solvates or hydrates, in an amorphous form.

19. Pharmaceutical preparations according to claims 13 to 18, wherein the formulation additionally comprises 1 to 25 wt.-% of hydrochlorothiazide.

## Amended claims

### Amended claims in accordance with Rule 137(2) EPC.

**1.** A process for the preparation of adsorbates of candesartan cilexetil and/or of its hydrates, wherein one starts from a solution consisting of candesartan cilexetil and/or its hydrates in at least one organic solvent, disperses in it an adsorbing material and removes the solvent and wherein the absorbing materials are selected in particular from the group of carbohydrates and related polyols, including celluloses, cellulose derivatives, sugars, sugar derivatives, cyclodextrins, starches, starch derivatives, polydextroses, or mixtures thereof.

**2.** The process according to claim 1, wherein the adsorbates contain the active ingredient, or its hydrates, in an amorphous form.

**3.** The process according to claim 2, wherein the weight ratio of active ingredient to adsorbing material is set in the range from 5:1 to 1:10, more particularly in the range from 2:1 to 1:2.

**4.** The process according to claim 1, wherein the weight ratio of active ingredient to adsorbing material is set in the range from 5:1 to 1:10, more particularly in the range from 2:1 to 1:2.

**5.** The process according to claim 1, wherein a single organic solvent is used or mixtures of solvents, these organic solvents being selected from the group of lower aliphatic ketones, alkanols, ethers, esters, aromatic and aliphatic hydrocarbons, amides, halogenated hydrocarbons, nitriles, and dialkyl sulfoxides.

**6.** The process according to claim 5, wherein the solvent is selected from acetone, methyl ethyl ketone, methanol, ethanol, isopropanol, n-propanol, 1-butanol, 2-butanol, t-butanol, diethyl ether, dimethyl ether, tetrahydrofuran, dioxane, diisopropyl ether, methyl acetate, ethyl acetate, n-propyl acetate, isopropyl acetate, toluene, xylene, mesitylene, petroleum ether, hexane, cyclohexane, heptane, dichloromethane, chloroform, ethylene dichloride, dimethyl formamide, dimethyl acetamide, or acetonitrile as well as mixtures of the aforementioned solvents.

**7.** The process according to claim 5, wherein acetone, toluene or dichloromethane are selected as solvents.

**8.** The process according to claim 1, wherein a solution of the active ingredient is used which is obtained in the final step of the candesartan cilexetil synthesis.

**9.** Adsorbates of candesartan cilexetil and its hydrates obtainable through the process according to claims 1 to 8.

**10.** Adsorbates of candesartan cilexetil and its hydrates according to claim 9, wherein the active ingredient or its pharmaceutically acceptable salts or derivatives and their hydrates are adsorbed in an amorphous form.

**11.** Adsorbates of candesartan cilexetil and its hydrates according to claim 9, wherein the adsorbates contain the active ingredient, or a pharmaceutically acceptable salt or other derivative of it, or their hydrates, in a substantially amorphous or semi-crystalline form.

**12.** Pharmaceutical formulations with at least one active ingredient and optionally further, pharmaceutically acceptable adjuvants, wherein the formulation contains a candesartan cilexetil adsorbate according to claims 1 to 11 as active ingredient.

**13.** Pharmaceutical formulations according to claim 12, wherein the formulation is in the formulation of tablets, capsules, pellets, and granules that are obtainable in a known way with common, pharmaceutically acceptable adjuvants.

**14.** Pharmaceutical formulations according to claims 12 and 13 in the form of tablets made by direct compressing of the components and **characterized by** a rapid release of the active ingredient.

**15.** Pharmaceutical formulations according to claims 12 to 14, wherein the portion of binder in the total mixture used to prepare the drug is between 0 and 50 wt.%.

**16.** Pharmaceutical preparations according to claims 12 to 15, wherein the portion of fillers and adjuvants in the total mixture of the drug preparation is from 1 to 99% by weight, preferably from 60 to 99% by weight.

**17.** Pharmaceutical preparations according to claims 12 to 16, wherein the adsorbates contain the active ingredient or a pharmaceutically acceptable salt or derivative of it, or their hydrates, in an amorphous form.

**18.** Pharmaceutical preparations according to claims 12 to 17, wherein the formulation additionally comprises 1 to 25 wt.-% of hydrochlorothiazide.
